(19) **Europäisches Patentamt / European Patent Office / Office européen des brevets**

(11) **EP 4 303 885 A1**

(12) **EUROPEAN PATENT APPLICATION**

(43) Date of publication:
**10.01.2024   Bulletin 2024/02**

(21) Application number: **22183166.2**

(22) Date of filing: **05.07.2022**

(51) International Patent Classification (IPC):
**G16H 50/20** *(2018.01)*

(52) Cooperative Patent Classification (CPC):
**G16H 50/20**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **F. Hoffmann-La Roche AG**
**4070 Basel (CH)**

(72) Inventors:
• **BERNTENIS, Nikolaos**
**4070 Basel (CH)**

• **DE VERA MUDRY, Cristina**
**4070 Basel (CH)**
• **GUTIERREZ BECKER, Benjamin**
**4070 Basel (CH)**
• **TECILLA, Marco**
**4070 Basel (CH)**

(74) Representative: **Mewburn Ellis LLP**
**Aurora Building**
**Counterslip**
**Bristol BS1 6BX (GB)**

(54) **PREDICTION OF THE PRESENCE OF A HISTOPATHOLOGICAL ABNORMALITY**

(57)    The present invention is directed towards the application of toxicogenomic methods to the detection and/or prediction of histopathological abnormalities in human or animal subjects based on clinical pathology data. It has been observed that reliable results may be obtained in the absence of any image data. A computer-implemented method of predicting the presence of a histopathological abnormality in an organ of a human or animal subject based on clinical pathology data comprises: receiving clinical pathology data obtained from the human or animal subject; applying an analytical model to the clinical pathology data, the analytical model configured to output a result indicative of the likelihood of the presence of a histopathological abnormality in the organ of the human or animal subject; and outputting the result.

Fig. 1

EP 4 303 885 A1

**Description**

TECHNICAL FIELD OF THE INVENTION

The present invention relates to computer-implemented methods of identifying a histopathological abnormality in a human or animal subject. Corresponding methods and systems are also provided.

BACKGROUND TO THE INVENTION

Drug development includes the safety assessment of test compounds in animals in order to determine their safety in humans. Preclinical toxicity studies consist of in-life, laboratory, molecular and *post mortem* assessments in animals such as rodents, dogs and nonhuman primates. Toxicogenomics studies are toxicology studies in which gene expression in certain organs is correlated with standard toxicological endpoints such as clinical pathology and histopathology, such as in Uehara *et al.* (2010)[1].

[1] Uehara, T., Ono, A., Maruyama, T., Kato, I., Yamada, H., Ohno, Y. and Urushidani, T. (2010), The Japanese toxicogenomics project: Application of toxicogenomics. Mol. Nutr. Food Res., 54: 218-227. https://doi.org/10.1002/mnfr.200900169

As in human pathology, there have been an increasing number of applications of digital and computational techniques in toxicologic pathology, see Abels *et al.* (2019)[2], Turner *et al.* (2020)[3], Turner *et al.* (2021)[4], and Mehrvar *et al.* (2021)[5].

[2] Abels, Esther, et al. (2019) "Computational pathology definitions, best practices, and recommendations for regulatory guidance: a white paper from the Digital Pathology Association." The Journal of pathology 249.3 (2019): 286-294.

[3] Turner, Oliver C., et al. "Society of toxicologic pathology digital pathology and image analysis special interest group article*: Opinion on the application of artificial intelligence and machine learning to digital toxicologic pathology." Toxicologic pathology 48.2 (2020): 277-294.

[4] Turner, Oliver C., et al. "Mini Review: The Last Mile-Opportunities and Challenges for Machine Learning in Digital Toxicologic Pathology." Toxicologic pathology 49.4 (2021): 714-719

[5]); Mehrvar S, Himmel LE, Babburi P, Goldberg AL, Guffroy M, Janardhan K, Krempley AL, Bawa B. Deep learning approaches and applications in toxicologic histopathology: Current status and future perspectives. J Pathol Inform [serial online] 2021 [cited 2021 Nov 16];12:42. Available from: https://www.jpathinformatics.org/text.asp?2021/12/1/42/329733

These new techniques will have a huge impact on the timelines of histopathologic evaluation. In addition it will help improve the quality of histopathology data. improve the quality, reproducibility and rigour of histopathology data; uniformly set thresholds for morphological changes in the control tissues; and reduce timelines. Computational pathology is not limited to the detecting of lesions or morphological patterns of lesions, but also involves the integration, complex analysis and interpretation of a broad array of assays for the diagnosis, treatment and prognosis of disease.

SUMMARY OF THE INVENTION

The present invention is directed towards the application of toxicogenomic methods to the detection and/or prediction of histopathological abnormalities in human or animal subjects based on clinical pathology data. Crucially, it has been observed that reliable predictions may be made obtained in the absence of any image data. Accordingly, a first aspect of the present invention provides a computer-implemented method of predicting the presence of a histopathological abnormality in an organ of a human or animal subject based on clinical pathology data, the computer-implemented method comprising: receiving clinical pathology data obtained from the human or animal subject; applying an analytical model to the clinical pathology data, the analytical model configured to output a result indicative of the likelihood of the presence of a histopathological abnormality in the organ of the human or animal subject; and outputting the result.

The term "predicting" may refer to determining at least one numerical or categorical value indicative of the presence of the histopathological abnormality.

The term "subject" as used herein, typically, relates to mammals, but could refer to other classes of animal. The subject may suffer from or shall be suspected to suffer from a disease, i.e. it may already show some or all of the negative symptoms associated with the said disease. In the present application, the organ of the human or animal subject is preferably the liver. However, the computer-implemented method is equally applicable to other organs such as the kidney. There are various kinds of histopathological abnormalities, the presence of which may be detected using the computer-implemented method of the first aspect of the invention. However, in a preferred case, the histopathological abnormality is a lesion.

As mentioned previously, it has been observed that reliable predictions may made in the absence of image data. Thus, by using the computer-implemented method of the present invention, it is possible reliably to predict the presence of histopathological abnormalities in organs of human or animal subjects without the need for the invasive or costly procedures which are required to obtain histopathological slide images. For completeness, it is prudent to state that in preferred implementations of the present invention, the clinical pathology data does not include image data. Herein, "image data" refers to data, such as electronic data, which is representative of an image of a region of the organ in question. The image may be a photograph of a histopathological slide or may have been obtained using a range of well-known medical imaging techniques.

Alternatively put, in preferred implementations of the present invention, the analytical model may be run only on clinical pathology data. In the context of the present application, "clinical pathology data" is used to refer to data which may be obtained non-invasively, and generally relates to the presence and amount of one or more analytes in a bodily fluid of the human or animal subject. The bodily fluid may include a sample of tissue/organ of a subject, and/or of a product produced by a tissue/organ of a subject. A product produced by a tissue/organ of a subject may e.g. be a product of secretion (e.g. a glandular secretion, milk, colostrum, tears, saliva, sweat, cerumen, mucus), sputum, semen, vaginal/cervical fluid, blood (plasma, serum), cerebrospinal fluid (CSF), a product of excretion, faeces, or urine, skin or hair.

The clinical pathology data may include measurements of the concentrations of one or more analytes in a bodily fluid of the human or animal subject. The analytes may comprise one or more biomarkers. Herein, the term "biomarker" refers to A biological molecule found in blood, other body fluids, or tissues that is a sign of a normal or abnormal process, or of a condition or disease. In some cases, the clinical pathology data may comprise measurements of concentrations of one or more analytes in one or more bodily fluids. For example, a first subset of the clinical pathology data may comprise measurements of concentrations of one or more analytes in a first bodily fluid, and a second subset of the clinical pathology data may comprise measurements of concentration of one or more analytes in second bodily fluid. This can, of course, be generalized to a general plurality of bodily fluids.

The one or more biomarkers may comprise one or more of the following: liver injury biomarkers, muscular injury biomarkers, and renal injury biomarkers. Herein, the term "injury biomarker" is used to refer to those biomarkers which are present in higher concentrations when an abnormality or other injury is present in the organ or tissue in question.

The analytes may also comprise one or more electrolytes.

The clinical pathology data may comprise a ratio of a concentration of a first analyte to a concentration of a second analyte (or *vice versa*). The clinical pathology may include a plurality of such ratios. In particular, the clinical pathology data may comprise a ratio of a concentration of albumin in the bodily fluid of the human or animal subject to a concentration of globulin in the bodily fluid of the human or animal subject. Alternatively, and for completeness, the clinical pathology data may comprise a ratio of a concentration of globulin in the bodily fluid of the human or animal subject to a concentration of albumin in the bodily fluid of the human or animal subject. This ratio is generally used to assist clinicians in identifying the cause of a change in protein levels in a bodily fluid of a user.

The liver injury biomarkers may comprise one or more of bilirubin; aspartate aminotransferase[6]; gamma glutamine transferase[7]; alanine aminotransferase[8]; and lactate dehydrogenase. In preferred cases, the liver injury biomarkers may comprise all of bilirubin; aspartate aminotransferase; gamma glutamine transferase; alanine aminotransferase; and lactate dehydrogenase. The liver injury biomarkers may further comprise one or more of the following: albumin; alkaline phosphatase[9]; cholesterol; globulin; glucose; protein; and triglycerides.

[6] This may also be referred to as "glutamate oxaloacetate transaminase".

[7] This may also be referred to as "gamma-glutamyl transpeptidase".

[8] This may also be referred to as "glutamate pyruvate transaminase".

[9] This may also be referred to as "serum alkaline phosphatase" or "plasma alkaline phosphatase".

In preferred cases, the electrolytes may comprise potassium. The electrolytes may further comprise one or more of the following: calcium; chloride; phosphate; and sodium.

In preferred cases, the muscular injury biomarkers may comprise creatinine kinase. A high level of creatinine kinase in the blood is generally indicative of recent muscle damage.

The renal injury biomarkers may include one or more of the following: creatinine, and urea nitrogen. These biomarkers are generally used to investigate kidney function.

In a particularly preferred implementation of the first aspect of the invention, the clinical pathology data comprises: a ratio of a concentration of albumin in the bodily fluid of the human or animal subject to a concentration of globulin in the bodily fluid of the human or animal subject, and a concentration of each of the following in the bodily fluid of the human or animal subject: bilirubin; aspartate aminotransferase; gamma glutamine transferase; alanine aminotransferase; lactate dehydrogenase; potassium; and creatinine kinase.

Having discussed in detail the nature of the clinical pathology data, we now set out more information about the analytical model. When applied to the clinical pathology data, the analytical model is configured to output a result indicative of the likelihood of the presence of a histopathological abnormality in the organ of the human or animal subject. Herein, the term "analytical model" may refer to a mathematical model configured for predicting at least one target variable for at least one state variable. The term "target variable" may refer to a clinical value which is to be predicted. The target variable value which is to be predicted may depend on the disease or condition whose presence or status is to be predicted. The target variable may be either numerical or categorical. For example, the target variable may be categorical and may be "positive" in case of presence of disease or "negative" in case of absence of the disease. The term "state variable" as used herein may refer to an input variable which can be filled in the prediction model such as data derived by medical examination and/or self-examination by a subject. The state variable may be determined in at least one active test and/or in at least one passive monitoring.

The target variable may be numerical such as at least one value and/or scale value. In this case, the state variable may comprise the clinical pathology data, and the target variable may comprise the indication of the likelihood of the presence of the histopathological abnormality in the organ of the human or animal subject.

The analytical model may be a regression model or a classification model. In the context of the present application, the term "regression model" may be used to refer to an analytical model, the output of which is a numerical value within a range. For example, the output of such a regression model in the present case may be a numerical value corresponding to a likelihood or probability of the presence of a histopathological abnormality in the organ of the human or animal subject. In the context of the present application, the term "classification model" may be used to refer to an analytical model, the output of which is a binary classification or score indicative of the presence or absence of a histopathological abnormality in the organ of the human or animal subject.

Specifically, the analytical model may be a machine-learning model trained to output a result indicative of the likelihood of the presence of a histopathological abnormality in the organ of the human or animal subject based on an input comprising clinical pathology data obtained from the human or animal subject. The machine-learning model may be a regression model or a classification model, as defined previously. The machine-learning model is preferably trained using supervised learning. Accordingly, in preferred implementations, the machine-learning model is a random forest model or a gradient boosting model. Herein, when we refer to a machine-learning model as a "random forest model", we mean that the machine-learning model has been trained using a random forest algorithm. Similarly, when we refer to a machine-learning model as a "gradient boosting model", we mean that the machine-learning model has been trained using a gradient boosting algorithm.

A random forest algorithm is a supervised learning algorithm which combines the output of multiple decision trees in order to reach a single result. When a random forest algorithm is used for regression, the output may comprise the mean or average prediction of the individual decision trees. When a random forest is used for classification, the output may comprise the class selected by the most individual decision trees. An algorithm such as those set out in Breiman (2001)[10], Ooka *et al.* (2021)[11], and Christodoulous *et al.* (2022)[12] may be used. Other examples of random forest algorithms are equally suitable.

[10] Breiman, L. Random Forests. Machine Learning 45, 5-32 (2001). https://doi.org/10.1023/A:1010933404324

[11] Ooka T, Johno H, Nakamoto K, et al Random forest approach for determining risk prediction and predictive factors of type 2 diabetes: large-scale health check-up data in Japan. BMJ Nutrition, Prevention & Health 2021;bmjnph-2020-000200. doi: 10.1136/bmjnph-2020-000200

[12] "Random forest classification algorithm for medical industry data" Christodoulos Vlachas, Lazaros Damianos, Nikolaos Gousetis, Ioannis Mouratidis, Dimitrios Kelepouris, Konstantinos-Filippos Kollias, Nikolaos Asimopoulos and George F Fragulis; SHS Web Conf., 139 (2022) 03008 DOI: https://doi.org/10.1051/shsconf/202213903008

A gradient boosting algorithm is a supervised learning algorithm which generates a prediction model in the form of an ensemble of weak prediction models such as decision trees. A gradient-boosted trees model is built in a stage-wise fashion as in other boosting methods, but it generalizes the other methods by allowing optimization of an arbitrary differentiable loss function. Examples of gradient boosting algorithms that may be used include *XGBoost*[13][14].

[13] https://arxiv.org/abs/1603.02754

[14] https://www.ncbi.nlm.nih.gov/pmc/articles/PMC6511546/

The analytical model is preferably configured to output a histopathological score indicative of the likelihood of the presence of a histopathological abnormality in the organ of the human or animal subject. This is the case regardless of whether the analytical model is a regression model or a classification model. In preferred cases, the histopathological score is a binary score (e.g. a "1" or a "0", although it will be appreciated that any binary scores can be used). Herein, a "binary score" is a score which may take two values only. Preferably, one of the values corresponds to a prediction of the presence of a histopathological abnormality, and the other values corresponds to a prediction of the absence of a histopathological abnormality.

A second aspect of the present invention provides a computer-implemented method of generating a machine-learning model configured to output a result indicative of the likelihood of the presence of a histopathological abnormality in the organ of the human or animal subject, the computer-implemented method comprising: receiving training data comprising, for each of a plurality of human or animal subjects: clinical pathology data and a histopathological score indicative of whether a histopathological abnormality is present in an organ of that human or animal subject; and training the machine-learning model using the received training data. Except where clearly incompatible, optional features set out above with regard to the first aspect of the invention apply equally well to the second aspect of the invention. Specifically, training the machine-learning algorithm may comprise using a random forest algorithm or a gradient boosting algorithm. In preferred cases, the analytical model the first aspect of the invention is a machine-learning model which is generated according to the computer-implemented method of the second aspect of the invention.

In some cases, the training data may comprise a plurality of subsets of training data, each set originating from a different source. In these cases, it may be desirable to standardize the training data, thereby reducing the variability between subsets of training data from different sources. Standardization of data may also reduce variability within a given subset of training data, too. Accordingly, before the step of training the machine-learning model, the computer-implemented method may further comprise normalizing the received training data. A variety of normalization techniques may be employed to do so, including logarithmic normalization, batch normalization, z-score normalization, and the use of a location-scale model.

It is known that the effectiveness of a machine-learning model may depend on the quality of training data which is used to train that machine-learning model. Accordingly, a computer-implemented method of generating a machine-learning model configured to output a result indicative of the likelihood of the presence of a histopathological abnormality in the organ of the human or animal subject may comprise: receiving training data comprising, for each of a plurality of human or animal subjects: clinical pathology data and a histopathological score indicative of whether a histopathological abnormality is present in an organ of that human or animal subject; training a first machine-learning model using training data in a first manner; training a second-machine learning model using the training data in a second manner; and selecting either the first trained machine-learning model or the second trained machine-learning model based on a performance metric.

In some cases, the first manner and second manner may refer to training the respective machine-learning models using different subsets of the training data. Specifically, the training data may comprise a first subset of training data and a second subset of training data. Then, the first machine-learning model may be trained using the first subset of training data, and the second machine-learning model may be trained using the second subset of training data. The first subset of training data and the second subset of training data may not overlap, i.e. there may be no individual subject who is represented in both the first subset of training data and the second subset of training data. Alternatively, the first subset of training data may partially overlap with the second subset of training data. The first subset of training data should not be identical to the second subset of training data, however.

In other cases, the first manner and second manner may refer to training the respective-machine learning models using different training algorithms. Specifically, the first machine-learning model may be trained using a first training algorithm, and the second machine-learning model may be trained using a second training algorithm. For example, the first training algorithm may be a random forest algorithm and the second training algorithm may be a gradient boosting algorithm. Alternatively, both the first training algorithm and the second training algorithm may be random forest training algorithms (albeit different ones), or both the first training algorithm and the second training algorithm may be gradient boosting algorithms (again, albeit different ones).

This may be further generalized: the computer-implemented may comprise training each of a plurality of machine-learning models each in a respective manner. Then, the final step may comprise selecting one of the plurality of trained machine-learning models based on a performance metric. "Each respective manner" may refer to e.g. a combination of a specific subset of training data and a training algorithm. In this way, when there is a variety of training data available it is possible to identify the subset of training data which leads to the best performance. Similarly, when there are a variety of training algorithms available for a given machine-learning model, this enables a user to select the best performing training algorithm.

We now discuss the performance metric. There are a variety of metrics which are available to evaluate the performance of machine-learning algorithms[15]. In preferred cases, the performance metric is the area under the receiver operating characteristic curve (commonly shortened to AUC or AUC-ROC), which quantifies the ability of a classification model accurately to perform classifications. Alternative performance metrics which may be used include F1 score, precision, recall, and classification accuracy.

[15] https://scikit-learn.org/stable/auto_examples/miscellaneous/plot_roc_curve_visualization_api.html

In order to obtain a performance metric it is often necessary to apply the trained machine-learning model to test data. In preferred cases, there is no overlap between the test data and the training data used to train to the model, in order to avoid training bias. Accordingly, the computer-implemented method may further comprise determining a first value of a performance metric for the first trained machine-learning model; and determining a second value of a performance metric for the second trained machine-learning model; and selecting the one of the first trained machine-learning model or the second trained machine-learning model which is associated with the better score. Herein, "better" is used to refer to the more favourable score, i.e. the score which indicates a better performance. In many cases, this will be the higher score, but in some cases a lower score may be indicative of better performance. In each case, determining a respective value of the performance metric may comprise applying the trained machine-learning model to test data, and determining the value of the performance metric based on the output of the machine-learning model. Preferably, each trained machine-learning model is applied to the same test data to ensure consistency between the various tests.

The previous aspects of the invention relate to computer-implemented methods. A further aspect of the invention may provide a system for predicting the presence of a histopathological abnormality in an organ of a human or animal subject based on clinical pathology data, the system comprising a processor configured to execute the computer-implemented methods of any previous aspect of the invention. The optional features set out with reference to the previous aspects of the invention also apply equally well to this system aspect of the invention, unless clearly incompatible.

A further aspect of the invention may provide a computer program comprising instructions which, when the program is executed by the computer or a processor thereof, cause it to execute the steps of the computer-implemented methods of the previous aspects of the invention. The optional features set out with reference to the previous aspects of the invention also apply equally well to this aspect of the invention, unless clearly incompatible. Yet a further aspect of the invention provides a computer-readable medium having stored thereon the computer program of the previous invention.

The invention includes the combination of the aspects and preferred features described except where such a combination is clearly impermissible or expressly avoided.

BRIEF DESCRIPTION OF THE DRAWINGS

Embodiments of the present invention will now be described with reference to the accompanying drawings, in which:

- Fig. 1 is a schematic of a system which may be used in implementations of the present invention.

- Fig. 2 is a flowchart illustrating the generation of a machine-learning model which may be used for the prediction of the presence or absence of a histopathological abnormality.

- Fig. 3 is a flowchart illustrating the use of a machine-learning model to predict the presence or absence of a histopathological abnormality.

DETAILED DESCRIPTION OF THE DRAWINGS

Aspects and embodiments of the present invention will now be discussed with reference to the accompanying figures. Further aspects and embodiments will be apparent to those skilled in the art. All documents mentioned in this text are incorporated herein by reference.

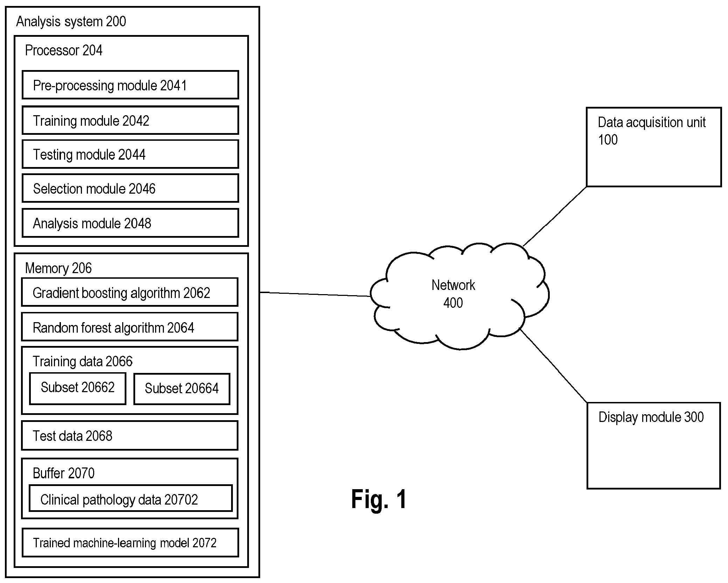

Fig. 1 shows an overall system 1 which may be used to execute computer-implemented methods according to the present invention. The system 1 includes a data acquisition unit 100, an analysis system 200, and a display module 300. The data acquisition unit 100, analysis system 200, and display module 300 are all interconnected via a network 400. The network 400 may be a wired network (such as a LAN or WAN) or a wireless network (such as a Wi-Fi network, the Internet, or a cellular network). In some cases, data acquisition unit 100, analysis system 200, and display module 300 may be connected via a plurality of networks 400 (not shown). For example, the acquisition module 100 and the analysis system 200 may be connected via a first network, the data acquisition unit 100, and the display module 300 may be connected via a second network, and the analysis system 200 and display module 300 may be connected via a third network. Other combinations are envisaged. Alternatively, some subsets of the data acquisition unit 100, analysis system 200, and display module 300 may be integrated with each other. For example, the data acquisition unit 100, analysis system 200, and display module 300 may all be integrated into a single system, such as a smartphone, desktop computer, laptop computer, or tablet. In some cases, the data acquisition unit 100 and the display module 300 may be client- or user-facing modules (i.e. they are accessible by an end-user or client of the system), whereas the analysis system 200 may be located remotely, e.g. on a server, such as a back-end server. Alternatively, the analysis system 200 may be located on the cloud so that the processing performed takes place outside a user device, on a server (or the like) having a higher computational capacity. Various other arrangements are envisaged.

In the context of the present application, the data acquisition unit 100 is a unit, which may include hardware and software components, which is adapted to obtain clinical pathology data from a human or animal subject. For example, a clinician or other scientist may obtain a sample of a bodily fluid from the human or animal subject in question, and use specialized hardware e.g. to generate the clinical pathology data using the obtained sample. Naturally, in the context of the present invention, the clinical pathology data is preferably electronic data, which contains information relating to the concentrations of various analytes in the bodily fluid of the human or animal subject in question, as outlined elsewhere in this patent application.

The analysis system 200 is where the bulk of the analysis which is central to the present invention is executed. The analysis system 200 comprises a processor 204 and a memory 208. The processor 204 comprises a plurality of modules. In the context of the present application, a module may be implemented in either hardware or software, and is adapted or configured to perform a particular function. For example, it may be used to refer to a physical component within the processor, or it may refer, for example, to a section of code which comprises instructions, which when executed by the processor 204, cause it to perform the function in question. Specifically, the processor 204 comprises a preprocessing module 2041, a training module 2042, a testing module 2044, a selection module 2046, and an analysis module 2048. The respective functions of each of these modules will be discussed later.

The memory 206 of the analysis system 200 may comprise persistent and temporary memory. In the specific implementation shown in Fig. 1, the memory 206 stores: a gradient boosting algorithm 2062, a random forest algorithm 2064, training data 2066 (which contains two subsets 20662, 20664), and test data 2068. The memory 206 also includes a buffer 2070, which may store, e.g. the clinical pathology data 20702 while processing is taking place (explained in more detail later).

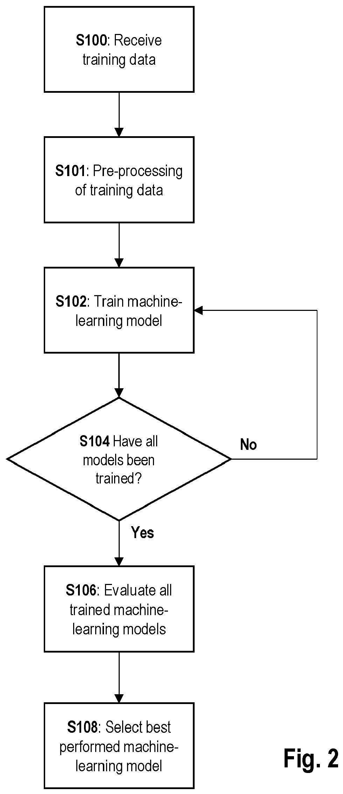

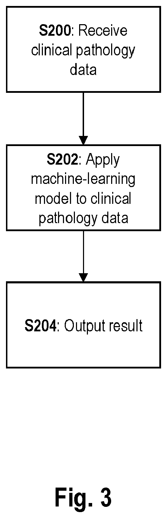

Having described the structure of the system 1, we now explain its operation, with reference to Figs. 2 and 3, which are high-level flowcharts illustrating, respectively, a computer-implemented method of generating a machine-learning model, and a computer-implemented method of using the machine-learning module to predict the presence of a histopathological abnormality in an organ of a human or animal subject based on clinical pathology data.

Fig. 2 illustrates a computer-implemented method of generating a machine-learning model which may be used to predict the presence of a histopathological abnormality in an organ of a human or animal subject based on clinical pathology data. In a first step, training data 2066 is received by the analysis system 200. The training data 2066 may be received from an appropriate source, for example from one or more external databases (not shown, but non-limiting examples are given in the experimental results section). Then, in step S101, pre-processing of the training data is performed by pre-processing module 2041 of the processor 204. This may comprise data normalization, as outlined elsewhere in this application, in order to reduce both inter- and intra-dataset variability. The overall aim of the training scheme shown in Fig. 2 is to train a plurality of machine-learning models in a plurality of different ways, evaluate the performance of each trained machine-learning model, and select the trained machine-learning model which has performed best. Accordingly, in step S102, a (first) machine-learning model is trained in a first way, using the training module 2042. This could mean several things: for example, a machine-learning model might be trained using a first type of training algorithm (e.g. the gradient boosting algorithm 2062, or the random forest algorithm 2064), or the machine-learning model could be trained using a first subset of data (e.g. subset 20662 or subset 20664). After the first machine-learning model has been trained, in step S104, it is determined whether all of the machine-learning models have been trained by the training module 2042. Here, "all" of the machine-learning models refers to training of a machine-learning model in each of the different ways in which it is to be trained. This may encompass, for example, training a machine-learning model using all combinations of subsets 20662, 20664 of training data 2066 and training algorithms (e.g. gradient boosting algorithm 2062 and random forest algorithm 2064). If it is determined in step S104 that not all machine-learning models have been trained, the computer-implemented method returns to step S102, and a further machine-learning model is trained in a different manner from the first. This iterative procedure continues until it is determined in step S104 that all machine-learning models have been trained. The computer-implemented method then moves on to step S106, in which the testing module 2044 evaluates the performance of each of the trained machine-learning models. Preferably, evaluation by the testing module 2044 involves determination or calculation of a performance metric, as outlined elsewhere in this application. The testing module 2044 may be configured to apply each trained machine-learning model to test data 2068, and to determine or calculate a performance metric based on the results of the application of the trained machine-learning model on the test data 2068. The test data 2068 may be a subset of the training data 2062 which was not used to train the machine-learning model (to avoid training bias). In the example of Fig. 2, the performance evaluation takes place after all of the machine-learning models have been trained. However, it is equally feasible that the performance evaluation of a given machine-learning model takes place (immediately) after it is generated. Alternatively, the performance evaluation process and the training processes may take place in parallel. Other arrangements are envisaged. After the performance of all of the trained machine learning models has been evaluated by the testing module 2044 in step S106, the computer-implemented method moves to step S108, in which the selection module 2046 selects the trained machine-learning model which has the best performance. This is preferably done according to the value of a performance metric. The selected machine-learning model 2072 may then be stored in the memory 206 of the analysis system 200.

Fig. 2 relates to the generation of a machine-learning model. Fig. 3 illustrates a computer-implemented method according to the first aspect of the invention, in which the presence or absence of a histopathological abnormality is predicted. In a first step S200, clinical pathology data 20702 is received. For example, the processor 204 of the analysis system 200 may receive the clinical pathology data 20702 from the data acquisition unit 100. For processing, the clinical pathology data 20702 may be stored in the buffer 2070 of the memory 206 of the analysis system 200. Then, in step S202, the analysis model 2048 may retrieve the trained machine-learning model 2072 from the memory 206 of the analysis system, and apply it to the clinical pathology data 20702. As discussed, the trained machine-learning model 2072 is preferably configured to output a binary histopathological score indicative of the presence or absence of a histopathological abnormality, based on the clinical pathology data 20702. Then, in step S204, the results are output. This may comprise the processor 204 transmitting the results to display module 300, whereupon they may be displayed on e.g. a clinician or other client.

EXPERIMENTAL RESULTS

We now set out the results of a study which demonstrate the effectiveness of computer-implemented methods according to the present invention. The experiments were focused on the detection of histopathological lesions in the livers of rats in toxicity studies. Data included in the experimental studies were from rats, for which both clinical pathology data and histopathology evaluation of the liver was available. Data from intermediate bleeding points that are not associated with histopathology, as well as data from recovery animals, were excluded.

Analysis was carried out using three data sets (i.e. three sets of training data):

-   **(i) TG-GATEs:** Online open-source dataset of toxicogenomics studies in rats, including whole slide images (20x) and associated clinical pathology data and histopathology findings. Include classic test items with well-characterized liver toxicity. The final dataset for liver cases was composed of 3,894 individual animals entries. Only male rats were present in this collection of data.

-   **(ii) Roche Toxicogenomics Initiative:** Dataset of rat studies the Toxicogenomics initiative that was conducted between 1999 and 2003. Test items used in these studies were classic hepatotoxicants or nephrotoxicants. The hepatotoxicants induced toxicity by the following mechanisms: direct-acting, steatotic, cholestatic, or immune-mediated. A few Roche compounds that were withdrawn due to clinical hepatotoxicity were also included. The dataset was composed of 1,768 entries, 1,705 males and 63 females.

-   **(iii) Roche toxicity studies:** 600 completed or SEND-like (standardization for exchange of non-clinical data) studies over the last 20 years. The dataset spanned over 20 years and consisted of 43,878 entries extracted from more than 600 studies. Of this larger dataset, a smaller section (referred to as (iv)) used in another project was extracted and tested separately. This extracted dataset was composed of 832 entries extracted from 34 studies.

As we explain later, different combinations of the above datasets were also tested to assess the impact on the AUC of the machine-learning models generated using the different datasets.

All datasets were visually inspected using the data analysis software *Spotfire (TIBCO).*

Earlier in this application, we referred to the use of data normalization to reduce variability. In these experiments, various different normalization techniques were tested to minimize intra- and inter-dataset (batch effect) variability, including: logarithmic normalization, batch normalization, z-score normalization, and location-scale model.

We now consider location-scale model reference ranges. Due to the diversity of the datasets in terms of both time frame and origin, normalization reference values were collected from the literature. Among various different location-scale models, the inventors selected the Chuang-Stein model[16]. The idea of the Chuang-Stein model is to normalize all values in relation to selected set of reference ranges:

$$value_{std} = (value - LLN) \cdot \frac{ULN_{std} - LLN_{std}}{ULN - LLN} + LLN_{std}$$

Herein:

-   *value* is the number to be normalized.

-   *ULN* and *LLN* are the upper and lower value in the dataset for the variable in question, respectively.

-   *ULN$_{std}$* and *LLN$_{std}$* are the upper and lower values in the standard reference list for the variable in question, respectively.

[16] https://www.lexjansen.com/phuse/2019/dh/DH05.pdf

The reference values were collected from the literature. Male reference values were considered in this specific context due to the higher prevalence of male subjects compared with female subjects in the datasets. The same reference ranges were applied to all examined datasets in order to obtain comparable data.

For all datasets, missing values were replaced by randomly generated values.

A histopathological score was calculated in two ways, depending on the dataset type.

-   For Roche Toxicogenomics Initiative studies, a senior toxicologic pathologist manually reviewed the histological diagnosis and severity, giving the final score. As a general approach, controls were set as normal (0), except if specific relevant findings were detected (e.g., liver necrosis). Treated animals were evaluated based on the histological score, with scores of 1 out of 5 or lower, set as normal (0). Samples with histological scores of 2 out of 5 or higher were set as pathological (1). An exception to this rule was liver glycogenosis, which was always considered normal.

- For Roche toxicity studies and TG-GATEs, a Python script analysed the severity of the finding reported by the pathologists and attributed an automatic score. The script was set to consider findings marked as 'Unremarkable,' 'Present,' or with a severity of 1 out of 5 as normal (represented in the table as 0). Results with higher severity were set as pathological (1). A junior toxicologic pathologist manually reviewed the automatic score to make it similar to the one given by the senior toxicologic pathologist for Toxicogenomics.

The data were then processed using:

1. Random forest and gradient boosting implementation in *Orange*[17].

2. Random forest implementation in Python using *scikit-learn*[18].

[17] https://orangedatamining.com/
[18] https://scikit-learn.org/stable/modules/generated/sklearn.ensemble.RandomForestClassifier.html

In order to avoid exposing the algorithm in question to the test information in advance, each dataset was split by study ID. The histopathological score was set as the target value.

The settings for *Orange* are shown below:

| | Number of trees | Learning rate | Depth of individual trees | Do not split subset smaller than | Fraction of training instances |
|---|---|---|---|---|---|
| **Random forest** | 10 | - | - | 5 | - |
| **Gradient boosting (scikit-learn)** | 100 | 0.100 | 3 | 2 | 1.00 |

The settings for the Python implementation were as follows:

| | Number of trees | Learning rate | Depth of individual trees | Do not split subset smaller than | Fraction of training instances |
|---|---|---|---|---|---|
| **Random forest** | 200 | *sklearn* default | *sklearn* default | *sklearn* default | *sklearn* default |

High intra- and inter-dataset variability were observed. Notably, high variability was noted between TG-GATEs and Toxicogenomics, while lower variability was observed between TG-GATEs and Roche's general studies and Roche Toxicogenomics Initiative and Roche toxicity studies. Intra-dataset variability was particularly high among Toxicogenomics studies.

After normalization, data distribution was similar among the three datasets, as shown in Tables 1, 2 and 3, in the annex to this patent application.

Among all types of data normalization, the location-scale model was an effective technique for normalizing clinical pathology data.

Results for each dataset taken alone processed in *Orange* are as follows, where the datasets have the same labels that were assigned above.

| Dataset | Model | AUC | CA[19] | F1[20] | Precision[21] | Recall |
|---|---|---|---|---|---|---|
| (i) | Random Forest | 0.817 | 0.731 | 0.730 | 0.747 | 0.731 |
| (i) | Gradient Boost | 0.819 | 0.754 | 0.754 | 0.758 | 0.754 |
| (iii) | Random Forest | 0.599 | 0.832 | 0.780 | 0.757 | 0.832 |

(continued)

| Dataset | Model | AUC | CA[19] | F1[20] | Precision[21] | Recall |
|---------|-------|-----|--------|--------|---------------|--------|
| (iii) | Gradient Boost | 0.622 | 0.847 | 0.799 | 0.803 | 0.847 |
| (iv) | Random Forest | 0.782 | 0.771 | 0.719 | 0.764 | 0.771 |
| (iv) | Gradient Boost | 0.750 | 0.785 | 0.779 | 0.776 | 0.785 |
| (ii) | Random Forest | 0.695 | 0.730 | 0.689 | 0.718 | 0.730 |
| (ii) | Gradient Boost | 0.707 | 0.710 | 0.697 | 0.693 | 0.710 |

[19] CA = classification accuracy
[20] F1= harmonic mean of the precision and recall.
[21] https://en.wikipedia.org/wiki/Precision and recall

When the Roche Toxicogenomics Initiative (ii) dataset was combined with other dataset and processed in *Orange*, results were as follows:

| Dataset | Model | AUC | CA | F1 | Precision | Recall |
|---------|-------|-----|-----|-----|-----------|--------|
| (i),(ii)+(iv) | Random Forest | 0.845 | 0.790 | 0.785 | 0.789 | 0.790 |
| (i)+(ii)+(iv) | Gradient Boost | 0.833 | 0.778 | 0.767 | 0.783 | 0.778 |
| (ii) + (iv) | Random Forest | 0.702 | 0.773 | 0.713 | 0.770 | 0.773 |
| (ii)+(iv) | Gradient Boost | 0.741 | 0.767 | 0.747 | 0.744 | 0.767 |

When we combined dataset (iv) with TG-GATEs (i), results in Orange were as follows:

| Dataset | Model | AUC | CA | F1 | Precision | Recall |
|---------|-------|-----|-----|-----|-----------|--------|
| (i)+(iv) | Random Forest | 0.831 | 0.749 | 0.743 | 0.764 | 0.749 |
| (i)+(iv) | Gradient Boost | 0.835 | 0.765 | 0.763 | 0.767 | 0.765 |

Roche toxicity dataset (iii) combined with TG-GATEs (i), and processed in Orange:

| Dataset | Model | AUC | CA | F1 | Precision | Recall |
|---------|-------|-----|-----|-----|-----------|--------|
| (i)+(ii) | Random Forest | 0.680 | 0.823 | 0.793 | 0.791 | 0.823 |
| (i)+(ii) | Gradient Boost | 0.703 | 0.834 | 0.795 | 0.812 | 0.834 |
| (ii)+(iii) | Random Forest | 0.683 | 0.823 | 0.793 | 0.791 | 0.823 |
| (ii)+(iii) | Gradient Boost | 0.704 | 0.834 | 0.794 | 0.811 | 0.834 |
| (i)+(ii)+(iii) | Random Forest | 0.716 | 0.819 | 0.795 | 0.795 | 0.819 |
| (i)+(ii)+(iii) | Gradient Boost | 0.737 | 0.829 | 0.799 | 0.812 | 0.829 |

Dataset (iv) was not included in the test since it is a subset of Roche toxicity dataset.
Results on 10-fold cross validation Random Forest:

| Dataset | Number of Subjects | AUC | Accuracy | F1 | Precision | Recall |
|---------|--------------------|-----|----------|-----|-----------|--------|
| (i) | 3897 | 0.82± 0.04 | 0.74±0.03 | 0.74± 0.03 | 0.81±0.02 | 0.67±0.09 |
| (iii) | 38195 | 0.68± 0.05 | 0.84±0.02 | 0.911 0.01 | 0.86±0.02 | 0.98±0.001 |
| (ii) | 1771 | 0.721 0.06 | 0.74±0.03 | 0.48± 0.13 | 0.6410.10 | 0.41±0.17 |
| (iv) | 833 | 0.75± 0.16 | 0.8210.10 | 0.411 0.16 | 0.69±0.22 | 0.3310.17 |

(continued)

| Dataset | Number of Subjects | AUC | Accuracy | F1 | Precision | Recall |
|---|---|---|---|---|---|---|
| (i)+ (ii)+(iv) | 8679 | 0.801 0.02 | 0.76$\pm$0.02 | 0.66$\pm$ 0.04 | 0.77$\pm$0.05 | 0.58$\pm$0.05 |

High variability in predictivity was observed between datasets, independently from sample numerosity. According to our preliminary results, Roche toxicity data performed better when combined with TG-GATEs. The combination of Roche toxicity and TG-GATEs datasets held an AUC of 0.82, which demonstrates that clinical pathology can be used for liver histopathology lesion detection.

GENERAL STATEMENTS

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

ANNEX – Table 1: TG-GATEs Summary

| Column | Avg | Min | Max | Median | StdDev | StdErr | Outliers | Range |
|---|---|---|---|---|---|---|---|---|
| Alanine Aminotransferase | 1.04 | 0.23 | 1.06 | 1.04 | 0.03 | 0.00 | 333 | 0.83 |
| Albumin | 46.92 | 37.00 | 58.00 | 46.77 | 2.82 | 0.05 | 22 | 21.00 |
| Albumin/Globulin | 2.64 | 1.77 | 3.07 | 2.66 | 0.22 | 0.00 | 12 | 1.30 |
| Alkaline Phosphatase | 0.98 | 0.30 | 1.03 | 0.99 | 0.03 | 0.00 | 97 | 0.73 |
| Aspartate Aminotransferase | 3.63 | 1.06 | 3.68 | 3.65 | 0.09 | 0.00 | 501 | 2.62 |
| Bilirubin | 3.53 | 1.20 | 3.59 | 3.56 | 0.11 | 0.00 | 142 | 2.39 |
| Calcium | 2.79 | 2.67 | 3.02 | 2.79 | 0.03 | 0.00 | 162 | 0.35 |
| Chloride | 100.72 | 97.00 | 104.93 | 100.64 | 0.56 | 0.01 | 103 | 7.93 |
| Cholesterol | 2.33 | 0.60 | 2.50 | 2.34 | 0.09 | 0.00 | 185 | 1.90 |
| Creatinine | 50.40 | 42.00 | 52.39 | 50.10 | 1.30 | 0.02 | 11 | 10.39 |
| Globulin | 1.99 | 1.60 | 2.30 | 1.99 | 0.07 | 0.00 | 14 | 0.70 |
| Gamma Glutamyl Transferase | 0.12 | 0.00 | 0.13 | 0.12 | 0.01 | 0.00 | 234 | 0.13 |
| Glucose | 7.47 | 3.90 | 9.10 | 7.48 | 0.34 | 0.01 | 160 | 5.20 |
| Lactate Dehydrogenase | 32.51 | 4.53 | 32.76 | 32.59 | 0.62 | 0.01 | 201 | 28.22 |
| Phosphate | 0.86 | 0.58 | 0.96 | 0.86 | 0.04 | 0.00 | 51 | 0.38 |
| Potassium | 5.54 | 3.88 | 6.11 | 5.55 | 0.18 | 0.00 | 95 | 2.23 |
| Protein | 59.65 | 55.50 | 65.60 | 59.71 | 0.92 | 0.01 | 133 | 10.10 |
| Sodium | 142.76 | 140.00 | 147.00 | 142.80 | 0.48 | 0.01 | 84 | 7.00 |
| Triglycerides | 2.22 | 0.40 | 2.42 | 2.26 | 0.15 | 0.00 | 134 | 2.02 |
| Urea Nitrogen | 11.77 | 5.01 | 12.05 | 11.80 | 0.26 | 0.00 | 136 | 7.04 |

ANNEX – Table 2: Roche Toxicogenomics Initiative Summary

| Column | Avg | Min | Max | Median | StdDev | StdErr | Outliers | Range |
|---|---|---|---|---|---|---|---|---|
| Alanine Aminotransferase | 1.06 | 0.23 | 1.06 | 1.06 | 0.04 | 0.00 | 190 | 0.83 |
| Albumin | 49.31 | 37.00 | 58.00 | 49.17 | 2.13 | 0.05 | 120 | 21.00 |
| Albumin/Globulin | 2.47 | 1.64 | 3.07 | 2.49 | 0.17 | 0.00 | 109 | 1.43 |
| Alkaline Phosphatase | 0.96 | 0.30 | 1.03 | 0.98 | 0.06 | 0.00 | 25 | 0.73 |
| Aspartate Aminotransferase | 3.65 | 1.06 | 3.69 | 3.67 | 0.13 | 0.00 | 205 | 2.62 |
| Bilirubin | 3.52 | 1.20 | 3.59 | 3.54 | 0.13 | 0.00 | 53 | 2.39 |
| Calcium | 2.78 | 2.37 | 3.02 | 2.78 | 0.06 | 0.00 | 196 | 0.65 |
| Chloride | 100.45 | 97.00 | 106.00 | 100.48 | 0.98 | 0.02 | 53 | 9.00 |
| Cholesterol | 2.41 | 0.60 | 2.51 | 2.41 | 0.07 | 0.00 | 40 | 1.92 |
| Creatinine | 49.91 | 26.53 | 53.05 | 50.03 | 1.66 | 0.04 | 186 | 26.53 |
| Globulin | 1.95 | 1.60 | 2.30 | 1.95 | 0.06 | 0.00 | 78 | 0.70 |
| Gamma Glutamyl Transferase | 0.13 | 0.00 | 0.13 | 0.13 | 0.01 | 0.00 | 179 | 0.13 |
| Glucose | 7.86 | 3.90 | 9.10 | 7.91 | 0.53 | 0.01 | 82 | 5.20 |
| Lactate Dehydrogenase | 32.32 | 4.53 | 32.76 | 32.46 | 1.09 | 0.03 | 55 | 28.22 |
| Phosphate | 0.83 | 0.58 | 0.96 | 0.83 | 0.04 | 0.00 | 46 | 0.38 |
| Potassium | 5.43 | 3.88 | 6.11 | 5.45 | 0.21 | 0.01 | 53 | 2.23 |
| Protein | 61.75 | 55.50 | 65.60 | 61.67 | 0.91 | 0.02 | 116 | 10.10 |
| Sodium | 141.04 | 140.00 | 147.00 | 140.89 | 0.91 | 0.02 | 236 | 7.00 |
| Triglycerides | 2.36 | 0.40 | 2.42 | 2.38 | 0.08 | 0.00 | 101 | 2.02 |
| Urea Nitrogen | 11.80 | 4.40 | 12.10 | 11.84 | 0.29 | 0.01 | 106 | 7.70 |

ANNEX – Table 3: Roche Toxicity Studies Summary

| Column | Avg | Min | Max | Median | StdDev | StdErr | Outliers | Range |
|---|---|---|---|---|---|---|---|---|
| Alanine Aminotransferase | 1.05 | 0.23 | 1.06 | 1.05 | 0.01 | 0.00 | 863 | 0.83 |
| Albumin | 57.99 | 37.00 | 58.00 | 58.00 | 0.52 | 0.00 | 20 | 21.00 |
| Albumin/Globulin | 3.02 | 1.64 | 3.07 | 3.02 | 0.02 | 0.00 | 256 | 1.43 |
| Alkaline Phosphatase | 1.03 | 0.30 | 1.03 | 1.03 | 0.02 | 0.00 | 1154 | 0.73 |
| Aspartate Aminotransferase | 3.67 | 1.06 | 3.69 | 3.67 | 0.03 | 0.00 | 1058 | 2.62 |
| Bilirubin | 3.58 | 1.20 | 3.59 | 3.59 | 0.03 | 0.00 | 591 | 2.39 |
| Calcium | 3.01 | 2.37 | 3.02 | 3.01 | 0.01 | 0.00 | 190 | 0.65 |
| Chloride | 103.57 | 97.00 | 106.00 | 103.55 | 0.26 | 0.00 | 1621 | 9.00 |
| Cholesterol | 2.41 | 0.60 | 2.51 | 2.46 | 0.11 | 0.00 | 176 | 1.92 |
| Creatinine | | 33.57008194 | 53.052 | | | | | |
| Globulin | 2.20 | 1.60 | 2.30 | 2.27 | 0.14 | 0.00 | 183 | 0.70 |
| Gamma Glutamyl Transferase | 0.13 | 0.00 | 0.13 | 0.13 | 0.00 | 0.00 | 979 | 0.13 |
| Glucose | 8.79 | 3.90 | 9.10 | 9.05 | 0.33 | 0.00 | 77 | 5.20 |
| Lactate Dehydrogenase | | 11.93010539 | 9.877557532 | | | | | |
| Phosphate | 0.95 | 0.58 | 0.96 | 0.95 | 0.01 | 0.00 | 627 | 0.38 |
| Potassium | 6.09 | 3.88 | 6.11 | 6.10 | 0.07 | 0.00 | 1432 | 2.23 |
| Protein | 63.82 | 56.77 | 65.60 | 63.72 | 1.45 | 0.01 | 4 | 8.83 |
| Sodium | 143.58 | 140.00 | 147.00 | 143.55 | 0.35 | 0.00 | 1864 | 7.00 |
| Triglycerides | 2.40 | 0.40 | 2.42 | 2.42 | 0.03 | 0.00 | 1355 | 2.02 |
| Urea Nitrogen | 11.91 | 4.40 | 12.10 | 11.91 | 0.12 | 0.00 | 305 | 7.70 |

**Claims**

1. A computer-implemented method of predicting the presence of a histopathological abnormality in an organ of a human or animal subject based on clinical pathology data, the computer-implemented method comprising:

    receiving clinical pathology data obtained from the human or animal subject;
    applying an analytical model to the clinical pathology data, the analytical model configured to output a result indicative of the likelihood of the presence of a histopathological abnormality in the organ of the human or animal subject; and
    outputting the result.

2. A computer-implemented method according to claim 1, wherein:
    the clinical pathology data comprises a ratio of a concentration of albumin in a bodily fluid of the human or animal subject to a concentration of globulin in the bodily fluid of the human or animal subject.

3. A computer-implemented method according to claim 1 or claim 2, wherein:
    the clinical pathology data comprises one or more concentration measurements of one or more respective liver injury biomarkers in a bodily fluid of the human or animal subject.

4. A computer-implemented method according to claim 3, wherein:
    the one or more liver injury biomarkers comprise: bilirubin; aspartate aminotransferase; gamma glutamine transferase; alanine aminotransferase; and lactate dehydrogenase.

5. A computer-implemented method according to any one of claims 1 to 4, wherein:
    the clinical pathology data comprises a measurement of a concentration of creatinine kinase in the bodily fluid of the human or animal subject.

6. A computer-implemented method according to any one of claims 1 to 5, wherein:
    the clinical pathology data comprises a measurement of a concentration of potassium in the bodily fluid of the human or animal subject.

7. A computer-implemented method according to any one of claims 1 to 6, wherein:
    the clinical pathology data does not include image data.

8. A computer-implemented method according to any one of claims 1 to 7, wherein:
    the analytical model is a machine-learning model trained to output a result indicative of the likelihood of the presence of a histopathological abnormality in the organ of the human or animal subject based on an input comprising clinical pathology data obtained from the human or animal subject.

9. A computer-implemented method according to claim 8, wherein:
    the machine-learning model is a random forest algorithm or a gradient boosting algorithm.

10. A computer-implemented method according to any one of claims 1 to 9, wherein:
    the analytical model is configured to output a histopathological score indicative of the likelihood of the presence of a histopathological abnormality in the organ of the human or animal subject.

11. A computer-implemented method according to claim 10, wherein:
    the histopathological score is a binary score.

12. A computer-implemented method according to any one of claims 1 to 11, wherein:
    the organ of the human or animal subject is the liver.

13. A computer-implemented method according to any one of claims 1 to 12, wherein:
    the histopathological abnormality is a lesion.

14. A computer-implemented method of generating a machine-learning model configured to output a result indicative of the likelihood of the presence of a histopathological abnormality in the organ of the human or animal subject, the computer-implemented method comprising:

receiving training data comprising, for each of a plurality of human or animal subjects: clinical pathology data and a histopathological score indicative of whether a histopathological abnormality is present in an organ of that human or animal subject; and

training the machine-learning model using the received training data.

15. A computer-implemented method according to any one of claims 1 to 13, wherein the analytical model is a machine-learning model trained according to the computer-implemented method of claim 14.

Fig. 1

S100: Receive training data

↓

S101: Pre-processing of training data

↓

S102: Train machine-learning model

↓

S104 Have all models been trained? — No

Yes

↓

S106: Evaluate all trained machine-learning models

↓

S108: Select best performed machine-learning model

Fig. 2

S200: Receive clinical pathology data

↓

S202: Apply machine-learning model to clinical pathology data

↓

S204: Output result

**Fig. 3**

Europäisches
Patentamt

European
Patent Office

Office européen
des brevets

# EUROPEAN SEARCH REPORT

Application Number

EP 22 18 3166

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/211787 A1 (CHILDRENS HOSPITAL MED CT [US]) 21 October 2021 (2021-10-21) * p. 598, 2nd column, last paragraph – p. 598, 3rd column, 2nd paragraph; p. 597, 3rd column, 2nd paragraph * ----- | 1-15 | INV. G16H50/20 |
| X | HE LILI ET AL: "Machine Learning Prediction of Liver Stiffness Using Clinical and T2-Weighted MRI Radiomic Data", AMERICAN JOURNAL OF ROENTGENOLOGY, vol. 213, no. 3, 30 September 2019 (2019-09-30), pages 592-601, XP055865944, US ISSN: 0361-803X, DOI: 10.2214/AJR.19.21082 * paragraphs 2-13, 116-123, 160-168, 38-53, 90 * ----- | 1-15 | |
| A | TAKEKI UEHARA ET AL: "The Japanese toxicogenomics project: Application of toxicogenomics", MOLECULAR NUTRITION & FOOD RESEARCH, vol. 54, no. 2, 28 February 2010 (2010-02-28), pages 218-227, XP055472505, DE ISSN: 1613-4125, DOI: 10.1002/mnfr.200900169 * the whole document * ----- | 1-15 | **TECHNICAL FIELDS SEARCHED (IPC)** G16H |

The present search report has been drawn up for all claims

| Place of search | Date of completion of the search | Examiner |
|---|---|---|
| Munich | 21 December 2022 | Wittke, Claudia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EP 4 303 885 A1

## ANNEX TO THE EUROPEAN SEARCH REPORT
## ON EUROPEAN PATENT APPLICATION NO.

EP 22 18 3166

This annex lists the patent family members relating to the patent documents cited in the above-mentioned European search report.
The members are as contained in the European Patent Office EDP file on
The European Patent Office is in no way liable for these particulars which are merely given for the purpose of information.

21-12-2022

| Patent document cited in search report | Publication date | Patent family member(s) | Publication date |
|---|---|---|---|
| WO 2021211787 A1 | 21-10-2021 | NONE | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **UEHARA, T. ; ONO, A. ; MARUYAMA, T. ; KATO, I. ; YAMADA, H. ; OHNO, Y. ; URUSHIDANI, T.** The Japanese toxicogenomics project: Application of toxicogenomics. *Mol. Nutr. Food Res.,* 2010, vol. 54, 218-227
- **ABELS ; ESTHER et al.** Computational pathology definitions, best practices, and recommendations for regulatory guidance: a white paper from the Digital Pathology Association. *The Journal of pathology,* 2019, vol. 249 (3), 286-294
- **TURNER ; OLIVER C. et al.** Society of toxicologic pathology digital pathology and image analysis special interest group article*: Opinion on the application of artificial intelligence and machine learning to digital toxicologic pathology. *Toxicologic pathology,* 2020, vol. 48 (2), 277-294
- **TURNER ; OLIVER C. et al.** Mini Review: The Last Mile-Opportunities and Challenges for Machine Learning in Digital Toxicologic Pathology. *Toxicologic pathology,* 2021, vol. 49 (4), 714-719
- **MEHRVAR S ; HIMMEL LE ; BABBURI P ; GOLDBERG AL ; GUFFROY M ; JANARDHAN K ; KREMPLEY AL ; BAWA B.** Deep learning approaches and applications in toxicologic histopathology: Current status and future perspectives. *J Pathol Inform,* 16 November 2021, vol. 12, 42, https://www.jpathinformatics.org/text.asp?2021/12/1/42/329733
- **BREIMAN, L.** Random Forests. *Machine Learning,* 2001, vol. 45, 5-32, https://doi.org/10.1023/A:1010933404324
- **OOKA T ; JOHNO H ; NAKAMOTO K et al.** Random forest approach for determining risk prediction and predictive factors of type 2 diabetes: large-scale health check-up data in Japan. *BMJ Nutrition, Prevention & Health,* 2021
- **CHRISTODOULOS VLACHAS ; LAZAROS DAMIANOS ; NIKOLAOS GOUSETIS ; LOANNIS MOURATIDIS ; DIMITRIOS KELEPOURIS ; KONSTANTINOS-FILIPPOS KOLLIAS ; NIKOLAOS ASIMOPOULOS ; GEORGE F FRAGULIS.** Random forest classification algorithm for medical industry data. *SHS Web Conf,* 2022, vol. 139, 03008, https://doi.org/10.1051/shsconf/202213903008